# EUROPEAN PATENT SPECIFICATION

(11) **EP 2 456 481 B1**
(45) Date of publication and mention of the grant of the patent: **23.11.2016**
(21) Application number: 10732600.1
(22) Date of filing: 08.07.2010
(51) Int. Cl.: A61L 31/08

(54) **MEDICAL DEVICES HAVING AN INORGANIC COATING LAYER FORMED BY ATOMIC LAYER DEPOSITION**
MEDIZINISCHE VORRICHTUNG MIT EINER ANORGANISCHEN BESCHICHTUNG MITTELS ATOMLAGENABSCHEIDUNG
DISPOSITIFS MÉDICAUX AYANT UNE COUCHE DE REVÊTEMENT INORGANIQUE FORMÉE PAR DÉPÔT DE COUCHES ATOMIQUES

(30) Priority: 24.07.2009 US 228264 P
(43) Date of publication of application: 30.05.2012
(73) Proprietor: Boston Scientific Scimed, Inc., Maple Grove, MN 55311 (US)
(72) Inventor: FLANAGAN, Aiden, Kilcolgan County Galway (IE); WEBER, Jan, NL-6228 GJ Maastrich (NL)
(74) Representative: Peterreins Schley
(86) International application number: PCT/US2010/041348
(87) International publication number: WO 2011/011207

(56) References cited:
- WO-A1-2009/091384
- WO-A1-2010/014510
- WO-A2-2009/132176
- US-A1- 2008 124 373
- US-A1- 2009 118 821
- DATABASE WPI Week 200916 Thomson Scientific, London, GB; AN 2009-B46586 XP002625586, YANG S J: "Stent for expanding blood vessel, comprises multiple pores and titanium oxide layer with superior stability, where pores are formed on surface of stent", & KR 2008 025 870 A (YANG S J) 24 March 2008 (2008-03-24)

## Description

### Technical Field

The present invention relates to medical devices, and in particular, medical devices having an inorganic coating.

### Background

The use of inorganic coatings on stents may improve the biocompatibility of medical devices. For example, inorganic coatings may reduce the thrombogenicity, tissue irritation, or tissue inflammation that may be associated with the implantation of stents into a blood vessel. However, there are various problems sometimes associated with the use of inorganic coatings on stents. Such problems may include, for example, cracking or delamination of the coating during stent deployment, the need for high process temperatures, lack of substrate adhesion, lack of flexibility, lack of uniformity, lack of coating process reliability, or lack of options for batch processing. As such, there is a need for improved inorganic coatings on medical devices such as stents.

### Summary

The present disclosure provides stents having an improved inorganic coating layer as characterised in claim 1. For example, inorganic coating layers of the present disclosure may be resistant to cracking and/or delamination. In another example, the inorganic coating layers of the present disclosure may be highly conformal and/or highly uniform in thickness. In another example, the inorganic coating layers of the present disclosure may be applied by batch processing to increase manufacturing efficiency. In another example, inorganic coating layers of the present disclosure may be used for controlling the release of a therapeutic agent.

In one embodiment, the present disclosure provides a medical device having a coating, the coating comprising: an inorganic coating layer comprising an inorganic material, wherein the inorganic coating layer has a thickness of less than 30 nm.

In another embodiment, the present disclosure provides a method of coating a medical device, comprising: providing a medical device having a coating of therapeutic agent; and depositing an inorganic coating layer over the therapeutic agent by atomic layer deposition.

In another embodiment, the present disclosure provides a method of medical treatment comprising: providing a medical device having a coating comprising an inorganic coating layer, the inorganic coating layer comprising an inorganic material; implanting the medical device into the patient's body; and deforming the medical device without substantially cracking or delaminating the inorganic coating layer.

In another embodiment, the present disclosure provides a stent having a luminal surface, an exterior surface, and a coating, the coating comprising: an inner inorganic coating layer disposed over the luminal surface of the stent; and an external inorganic coating layer disposed over the external surface of the stent; wherein the thickness of the inner coating layer and the thickness of the external coating layer are substantially the same or differ by less than 20%, In some cases, the coating is conformal over the stent. In some cases, the inner inorganic coating layer has a thickness of less than 30 nm.

In another embodiment, the present disclosure provides a stent having a coating, the coating comprising: a therapeutic agent; and an inorganic coating layer disposed over the therapeutic agent, wherein the inorganic coating layer is formed by atomic layer deposition. In some cases, the inorganic coating layer is porous to the therapeutic agent. In some cases, the medical device further comprises a base coating layer disposed beneath the inorganic coating layer, wherein the base coating layer is in contact with the therapeutic agent or disposed between the surface of the medical device and the therapeutic agent. In some cases, the inorganic coating layer comprises aluminum oxide and the base coating layer comprises titanium oxide.

### Brief Description of the Drawings

FIGS. 1A - E illustrate an example of how a coating can be formed by atomic layer deposition.
FIGS. 2A - F illustrate how an aluminum oxide coating may be formed on a medical device by atomic layer deposition.
FIGS. 3A - C show a stent having a coating deposited by atomic layer deposition. FIG. 3A shows a perspective view of the stent. FIG. 3B shows an end view of the stent. FIG. 3C shows a close-up view of the corners between the stent struts.
FIG. 4A is a microscopic image of a 5 nm thick titanium oxide coating on a coronary artery stent. FIG. 4B is a microscopic image of a 30 nm thick titanium oxide coating on a coronary artery stent.
FIGS. 5A and B show an example of how an inorganic coating layer may be deposited over a therapeutic agent.
FIG. 6 shows an image of paclitaxel particles on a stent.
FIG. 7 shows a paclitaxel-coated stent with a 20 nm Al₂O₃ coating layer over the drug.
FIG. 8 shows a paclitaxel-coated stent with a 20 nm SiO₂ coating layer over the drug.
FIG. 9 shows a paclitaxel-coated stent with a 5 nm TiO₂ coating layer over the drug.
FIG. 10 shows the results of an experiment testing the barrier characteristics of an inorganic coating layer deposited by atomic layer deposition.
FIG. 11 shows a multilayered coating on a medical device.
FIGS. 12A - C show an example of how a base coating layer, a therapeutic agent, and an inorganic coating layer may be deposited on a medical device.

### Detailed Description

Medical devices of the present disclosure have a coating that comprises one or more inorganic coating layers. An inorganic coating layer of the present disclosure may be continuous or discontinuous (e.g., the coating layer may be patterned, or distributed as islands or particles). In certain embodiments, the one or more inorganic coating layers are formed by a self-limiting deposition process. In a self-limiting deposition process, the growth of the coating layer stops after a certain point (e.g., because of thermodynamic conditions or the bonding nature of the molecules involved), even though sufficient quantities of deposition materials are still available. For example, the coating layer may grow in a layer-by-layer process where the growth of each monolayer is completed before the next monolayer is deposited.

Various types of self-limiting deposition processes suitable for making an inorganic coating layer may be used. Examples of self-limiting deposition processes include atomic layer deposition (also known as atomic layer epitaxy), pulsed plasma-enhanced chemical vapor deposition (see Seman et al., Applied Physics Letters 90:131504 (2007)), molecular layer deposition, and irradiation-induced vapor deposition.

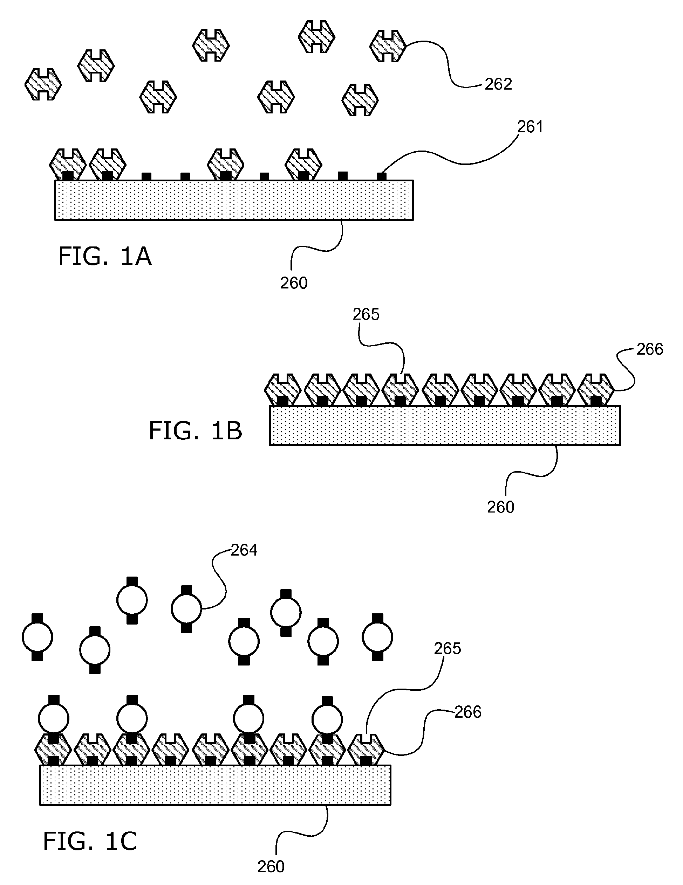

Atomic layer deposition is a gas-phase deposition process in which a coating is grown onto a substrate by self-limiting surface reactions. Atomic layer deposition is commonly performed using a binary reaction sequence, with the binary reaction being separated into two half-reactions. FIGS. 1A - E schematically illustrate an example of how a coating can be formed by atomic layer deposition using two sequential half-reactions. Referring to FIG. 1A, a substrate 260 with a surface having reactive sites 261 is placed inside a reaction chamber. In the first half-reaction, a first precursor species 262 in vapor phase is fed into the reaction chamber. First precursor species 262 is chemisorbed onto the surface of substrate 260 by reacting with reactive sites 261. As shown in FIG. 1B, the chemisorption of precursor species 262 proceeds until saturation of the surface, at which point the reaction self-terminates, resulting in a monolayer 266. Once this half-reaction is completed, additional reactant exposure produces no additional growth of monolayer 266. The reaction chamber is then purged of first precursor species 262. Monolayer 266 has reactive sites 265 for reacting with the next precursor material.

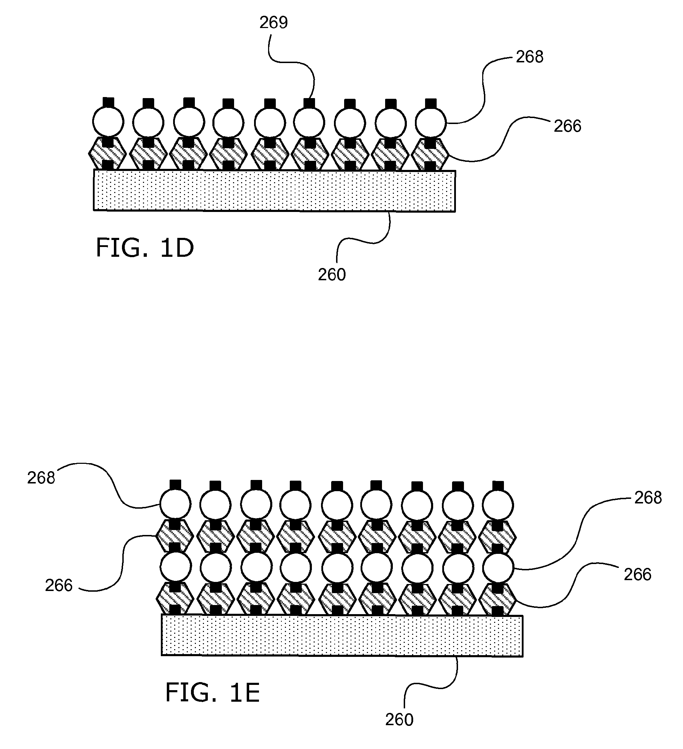

As shown in FIG. 1C, for the second half-reaction, a second precursor species 264 in vapor phase is fed into the reaction chamber. Second precursor species 264 reacts with reactive sites 265 on the surface of monolayer 266. As shown in FIG. 1D, the chemisorption of second precursor species 264 proceeds until saturation of monolayer 266, at which point the reaction self-terminates, resulting in another monolayer 268. The reaction chamber is then purged of second precursor species 264. The surface of monolayer 268 has reactive sites 269 capable of reacting with first precursor species 262, allowing additional reaction cycles until the desired coating thickness is achieved. For example, FIG. 1E shows substrate 260 having a series of monolayers 266 and 268 formed by several reaction cycles.

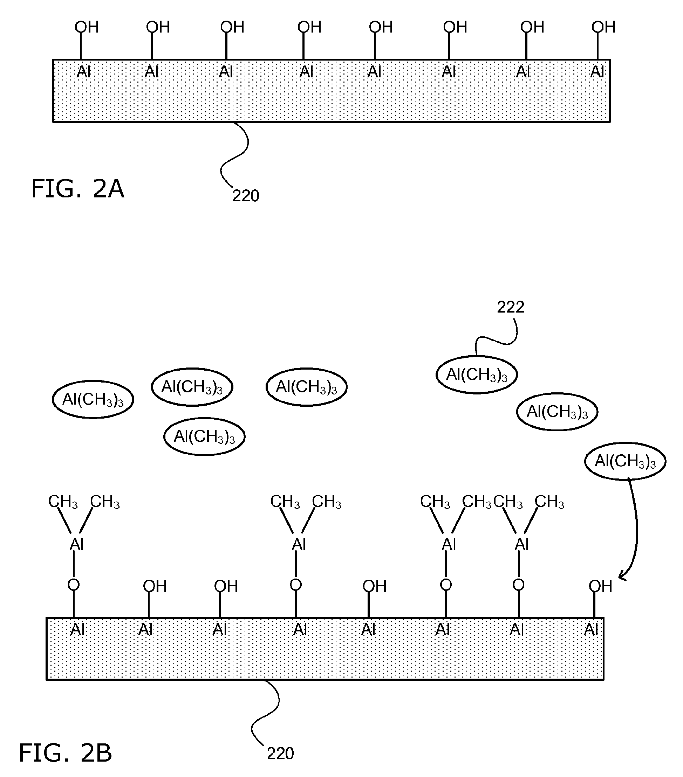

FIGS. 2A - F demonstrate how an aluminum oxide coating layer may be formed over a medical device by atomic layer deposition. The process involves the following two sequential half-reactions:
(A) :Al-OH + Al(CH₃)_{3(g)} → :Al-O-Al(CH₃)₂ + CH₄
(B) :Al-O-Al(CH₃)₂ + 2 H₂O → :Al-O-Al(OH)₂ + 2 CH₄
with :Al-OH and :Al-O-Al(CH₃)₂ being the surface species. These two half-reactions give the overall reaction :Al-OH + Al(CH₃)₃ + 2 H₂O → :Al-O-Al(OH)₂ + 3 CH₄.

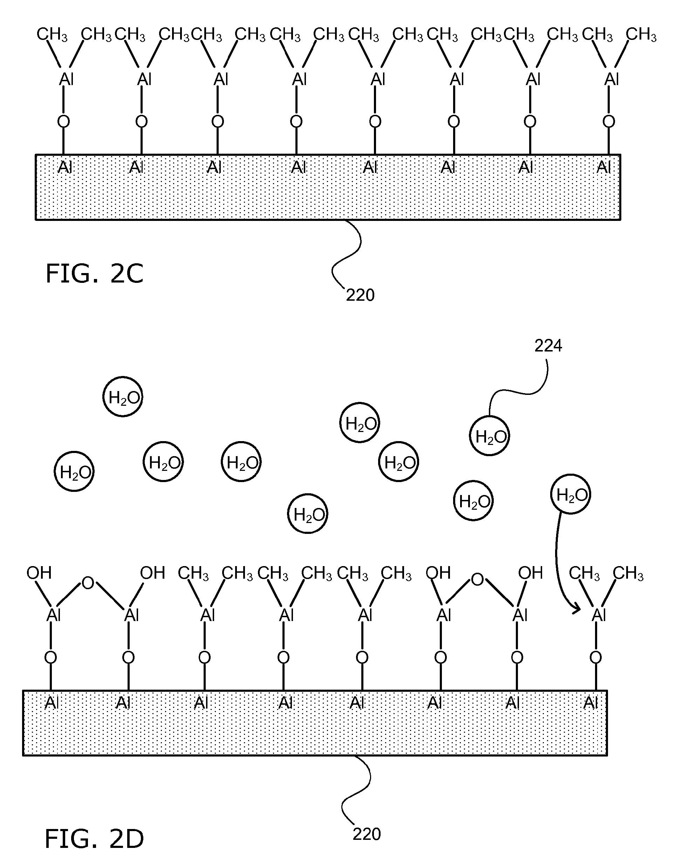

FIG. 2A shows a portion 220 of a medical device providing an aluminum surface having native hydroxyl groups. These native hydroxyl groups may be provided by pretreatment of the aluminum surface with water vapor. Referring to FIG. 2B, the medical device is placed inside a reaction chamber and Al(CH₃)₃ (trimethylaluminum) gas is introduced into the reaction chamber. The Al(CH₃)₃ molecules react with the native hydroxyl groups on the aluminum surface to form a methyl-terminated aluminum species. Referring to FIG. 2C, after all the native hydroxyl groups are reacted with Al(CH₃)₃, the reaction self-terminates, resulting in a monolayer of methyl-terminated aluminum. The reaction chamber is then purged of the excess Al(CH₃)₃ gas.

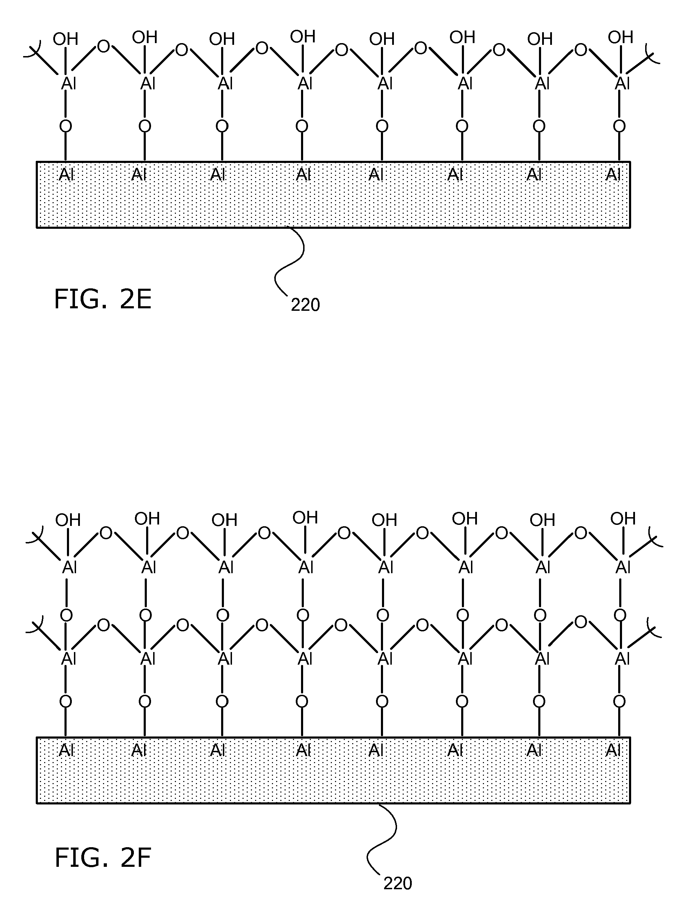

Next, water vapor is introduced into the reaction chamber. As shown in FIG. 2D, the water molecules 224 react with the dangling methyl groups on the new monolayer surface to form Al-0 bridges and surface hydroxyl groups. Referring to FIG. 2E, after all the methyl-terminated aluminum species are reacted with the water molecules 224, the reaction self-terminates, resulting in a monolayer of aluminum hydroxide species. This monolayer of aluminum hydroxide species has hydroxyl groups that are ready for the next cycle of exposure to trimethylaluminum. Referring to FIG. 2F, these reactions are repeated in a cyclic manner to form a coating of the desired thickness. This type of atomic layer deposition is available at Beneq (Vantaa, Finland).

Atomic layer deposition can be used to deposit numerous types of materials, including both inorganic and organic materials. For example, besides Al₂O₃, atomic layer deposition coating schemes have been designed for silica (SiO₂), silicon nitride (Si₃N₄), titanium oxide (TiO₂), boron nitride (BN), zinc oxide (ZnO), tungsten (W), and others. Also, it is known that an iridium oxide coating can be deposited by atomic layer deposition using an alternating supply of (ethylcyclopentadienyl)(1,5-cyclooctadiene)iridium and oxygen gas at temperatures between 230 to 290 °C. Other inorganic materials that could be deposited using atomic layer deposition include B₂O₃, Co₂O₃, Cr₂O₃, CuO, Fe₂O₃, Ga₂O₃, HfO₂, In₂O₃, MgO, Nb₂O₅, NiO, Pd, Pt, SnO₂, Ta₂O₅, TaNₓ, TaN,AlN, TiCrOₓ, TiN, VO₂, WO₃, ZnO, (Ta/Al)N, (Ti/Al)N, (Al/Zn)O, ZnS, ZnSe, ZrO, Sc₂O₃, Y₂O₃, Ca₁₀(PO₄)(OH)₂(hydroxylapatite), and rare earth oxides. Atomic layer deposition has also been used with organic materials, including 3-(aminopropyl) trimethoxysiloxane and polyimides, such as 1,2,3,5-benzenetetracarboxylic anhydride-4,4-oxydianiline (PMDA-ODA) and 1,2,3,5-benzenetetracarboxylic anhydride-1,6-diaminohexanc (PMDA-DAH).

Coating medical devices by atomic layer deposition can also allow for batch processing to improve manufacturing efficiency and/or process reliability. Multiple medical devices can be placed into a coating chamber to simultaneously coat the medical devices by atomic layer deposition. Also, because this may allow multiple medical devices to be subjected to the same deposition conditions, process reliability can be improved because substantially the same coating can be applied to each medical device.

By using a self-limiting deposition process to form the inorganic coating layer, the coating layer can have more uniformity in thickness across different regions of the medical device and/or a higher degree of conformality. The present invention may be useful in coating medical devices having a spatially challenging structure where coating uniformity may otherwise be difficult to achieve. For example, stents can present a challenging geometry for conventional line-of-sight coating techniques (such as spray coating). Stents coated by spray coating techniques will often have thinner coatings on the less accessible luminal surface (facing internally) as compared to the exterior surface (e.g., the coating on the luminal surface can be one-third the thickness of the coating on the exterior surface).

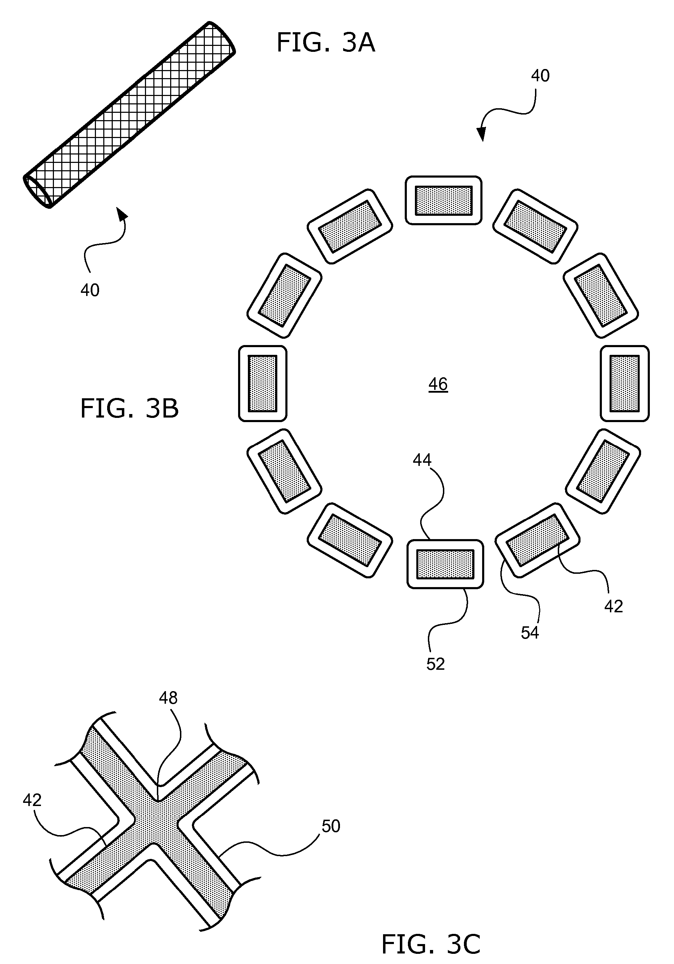

Referring to the embodiment shown in FIGS. 3A - C, a coronary artery stent 40 has an inorganic coating layer deposited by atomic layer deposition. FIG. 3A shows a perspective view of stent 40, which is formed of stent struts 42 in an open lattice configuration. As shown in the end view of FIG. 3B, stent 40 has an interior lumen 46 defined by stent struts 42. As also seen in this view, stent 40 has an inner coating layer 44 on the luminal side of stent 40 and an external coating layer 52 on the external side (i.e., abluminal) of stent 40. Atomic layer deposition of the coating layer can provide a more uniform coating thickness on the stent. As such, the thickness of inner coating layer 44 as compared to the thickness of the external coating layer 52 can differ, for example, by less than 20% of the thickness of the external coating layer (e.g., the inner coating layer may be thinner), or in some cases, can differ by less than 10%, or in some cases, can be substantially the same. Also, the sidewalls of stent struts 42 are also coated with sidewall coating layer 54. External coating layer 52, inner coating layer 44, and sidewall coating layer 54 together form a conformal coating around stent 40. The thickness of sidewall coating layer 54 as compared to the thickness of inner coating layer 44 or external coating layer 52 can differ, for example, by less than 20% of the thickness of the inner or external coating layer (e.g., sidewall coating layer 54 may be thinner), or in some cases, can differ by less than 10%, or in some cases, can be substantially the same.

Also, it has been demonstrated that very high aspect ratio structures (such as deep and narrow trenches or nanoparticles) can be coated uniformly by atomic layer deposition. Thus, certain embodiments in accordance with the present disclosure may allow for a more conformal coating on medical devices having a complex geometry. For example, in stents, the corners where the stent struts meet or join can present a coating challenge. With line-of-sight coating processes (e.g., spray coating), there may be a gap in coverage or disproportionately thin coatings at the corners. Alternatively, in liquid phase processes such as dip coating or sol-gel, the coating fluid may accumulate at the stent corners due to surface tension. This could result in the coating at the stent corners being disproportionately thicker than at the linear strut portions. Because these strut corners may be locations where the stent undergoes strain during stent expansion, coatings that are too thick at the corners are more likely to crack and/or delaminate during stent expansion.

FIG. 3C shows an expanded view of the open lattice configuration formed by stent struts 42. Stent struts 42 form corners 48 where different strut 42 meet. Inorganic coating layer 50 penetrates into and provides coverage at these corners 48. Thus, inorganic coating layer 50 may be conformal over stent 40. As used herein, "conformal" means that the coating layer follows the contours of the medical device geometry and continuously covers over substantially all the surfaces of the medical device. Coating layer 50 is also sufficiently thin and uniform to resist cracking and/or delamination at corners 48 during stent expansion.

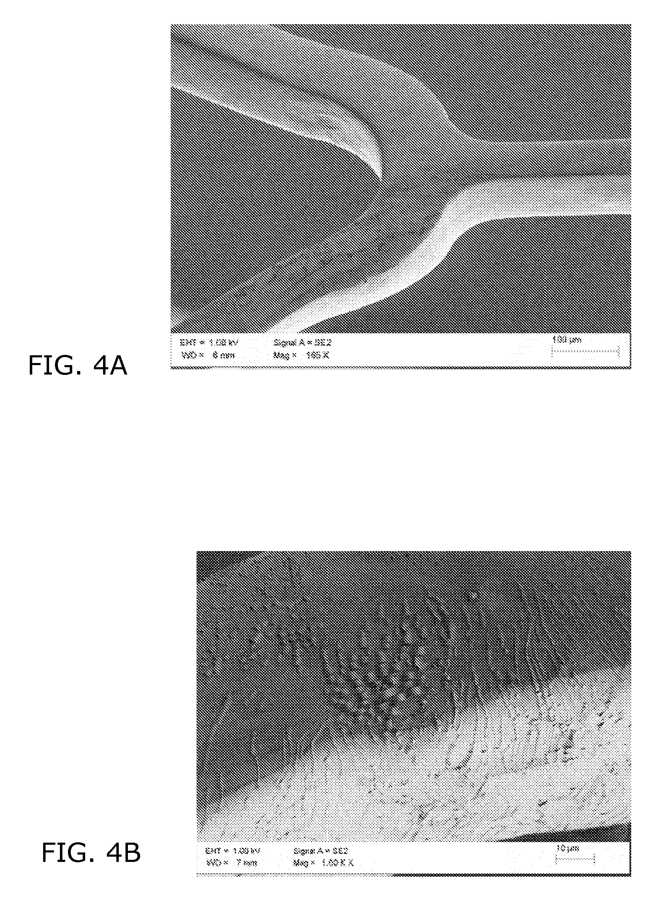

An inorganic coating layer in accordance with the present disclosure may have various thicknesses, depending upon the particular application. For FIGS. 4A and 4B, coronary artery stents were coated with titanium oxide by atomic layer deposition at 80 °C to a thickness of either 5 nm or 30 nm. FIG. 3A shows a microscopic image of the stent having the 5 nm thick titanium oxide coating, with the image taken after expansion of the stent. As seen here, there was no visible cracking or delamination of the titanium oxide coating. FIG. 3B shows a microscopic image of the stent having the 30 nm thick titanium oxide coating, with the image taken after expansion of the stent. As seen here, there was some cracking and delamination of the coating at high strain points after expansion of the stent. Based on these results, in some embodiments, the thickness of the inorganic coating layer is less than 30 nm, and in some cases, less than 20 nm. The inorganic coating layer may be as thin as 0.5 nm, but other thicknesses are also possible. Other types of coating processes, such as sol-gel techniques, may not be able to provide inorganic coatings of such uniform thinness.

Because the inorganic coating layers of the present invention can be resistant to cracking and/or delamination, the inorganic coating layers may be useful in coating medical devices that undergo deformation during deployment. For example, stents, stent grafts, catheters, balloon catheters, and guide wires can undergo significant deformation during deployment. In certain embodiments, the inorganic coating layer does not crack or delaminate despite deformation of the medical device during deployment in a patient's body. For example, a stent having an inorganic coating layer of the present invention may undergo expansion (e.g., with at least a 50% increase in diameter) during deployment of the stent without cracking or delamination of the inorganic coating layer.

Various properties of the inorganic coating layer, including its biocompatibility, crystal structure, porosity (e.g., nanoporosity), stability (e.g., degradability or dissolvability upon implantation), or substrate adhesion can be modified by selecting the coating layer material and/or deposition conditions. As mentioned above, inorganic coating layers in accordance with embodiments of the present disclosure may comprise various types of inorganic materials, including inorganic nitrides, inorganic oxides, or metals. Inorganic oxides include, for example, titanium oxide, aluminum oxide, silicon oxide, or zinc oxide.

Titanium oxide coatings are known to be very biocompatible and have low thrombogenicity, and because of its better corrosion resistance, it can be even more biocompatible than stainless steel. Titanium oxide coatings are also biostable and can serve as a permanent coating on an implantable medical device. The biocompatibility, porosity, surface interface, and/or corrosion resistance of titanium oxide coatings can also depend upon its crystal structure. In this regard, titanium oxide may exist in an amorphous or crystalline form. In atomic layer deposition, the crystalline anatase form of titanium oxide preferentially develops at relatively higher deposition temperatures (e.g., greater than 250 °C), whereas the amorphous form of titanium oxide preferentially develops at relatively lower deposition temperatures (e.g., less than 150 °C).

In comparison to a titanium oxide coating layer, an aluminum oxide coating layer can undergo degradation more quickly upon immersion in an aqueous solution or implantation in a patient's body. An aluminum oxide coating layer can also be more porous than a titanium oxide coating layer of the same thickness. Aluminum oxide can also be deposited at relatively lower deposition temperatures. For example, aluminum oxide may be deposited by atomic layer deposition at temperatures as low as about 50 °C using trimethylaluminum and water.

In certain embodiments, the coating on the medical device further comprises a therapeutic agent. An inorganic coating layer of the present invention is disposed over the therapeutic agent as a barrier layer for controlling the release of the therapeutic agent. The therapeutic agent may be distributed in a number of ways, including as a continuous layer or discontinuous layer (e.g., the therapeutic agent may be a patterned layer, or distributed as islands or particles). When an inorganic coating layer is deposited over the therapeutic agent, the deposition temperature may be selected to avoid or reduce heat degradation of the therapeutic agent. For example, a deposition temperature of less than 125 °C may be useful for preserving the therapeutic agent during the deposition process. Deposition temperatures as low as 50 °C may be used, but other deposition temperatures are also possible.

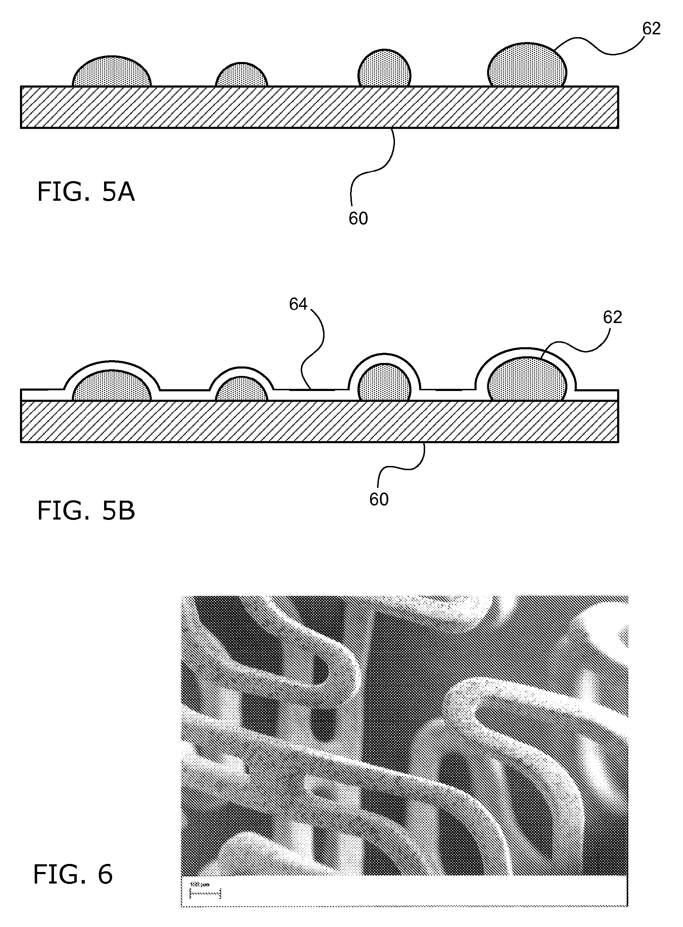

FIGS. 5A and B show an example of how an inorganic coating layer may be deposited over a therapeutic agent. FIG. 5A shows a portion 60 of a medical device. A coating of therapeutic agent is applied onto the portion 60 of the medical device. In this example, the therapeutic agent is applied in a liquid solution and upon drying, the therapeutic agent becomes distributed into particles 62. As an example, FIG. 6 shows an image of paclitaxel particles on a stent. Referring to FIG. 5B, an inorganic coating layer 64 is deposited over the medical device and therapeutic agent particles 62 by atomic layer deposition. As seen here, inorganic coating layer 64 conformally coats over the particles 62 of therapeutic agent. This can improve the adhesion of any particles 62 that are loosely bound to the surface. In an alternate embodiment, instead of particles 62, the therapeutic agent may be provided as a continuous layer.

Various properties of the inorganic coating layer 64 will affect the release rate of the therapeutic agent, such as the porosity and/or the degradability of inorganic coating layer 64. The porosity and/or degradability of inorganic coating layer 64 may depend upon its composition. For example, an inorganic coating layer formed of aluminum oxide, zinc oxide, or silicon oxide may be more porous or degrade more rapidly (e.g., within days or weeks after immersion in an aqueous solution or implantation in a patient's body) than a titanium oxide coating layer of the same thickness. In some cases, the inorganic coating layer degrades completely within 4 weeks after implantation of the medical device in a patient's body.

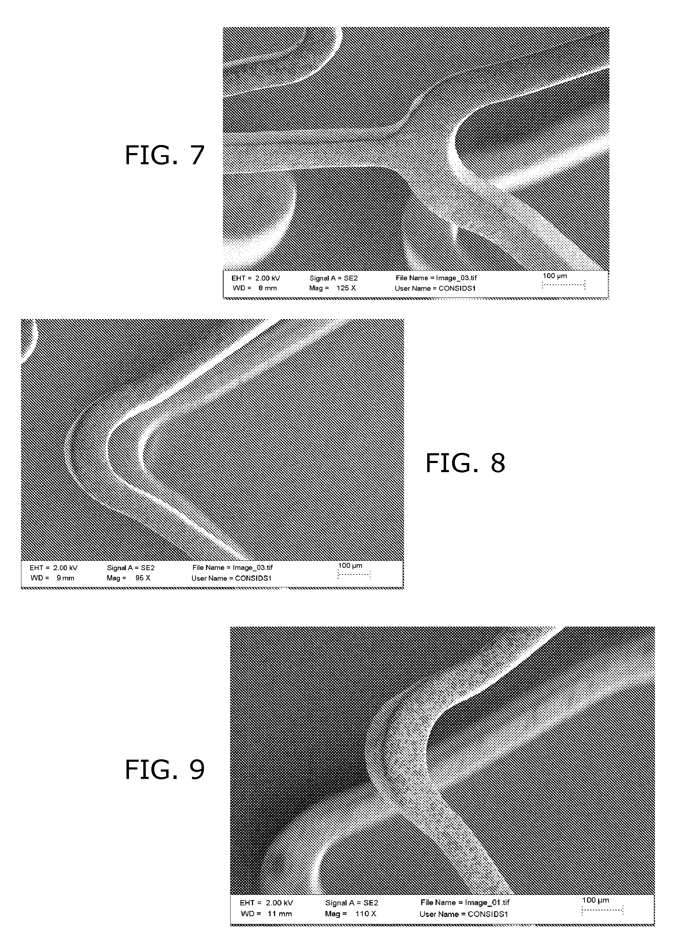

Further examples of embodiments in accordance with the present disclosure are shown in the images of FIGS. 7 - 9, which show inorganic coating layers formed on stents by atomic layer deposition. For FIG. 7, a metal stent was coated with paclitaxel particles and a 20 nm Al₂O₃ coating layer was deposited over the paclitaxel particles by atomic layer deposition at a temperature of 80 °C. The image in FIG. 7 was taken after crimping and expansion of the stent. As seen here, there was no visible delamination or cracking of the Al₂O₃ coating layer. For FIG. 8, a metal stent was coated with paclitaxel particles and a 20 nm SiO₂ coating layer was deposited over the paclitaxel particles by atomic layer deposition at a temperature of 75 °C. The image in FIG. 8 was taken after crimping and expansion of the stent. As seen here, there was no visible delamination or cracking of the SiO₂ coating layer. For FIG. 9, a stent was coated with paclitaxel particles and a 5 nm TiO₂ coating layer was deposited over the paclitaxel particles by atomic layer deposition at a temperature of 80 °C. The image in FIG. 9 was taken after crimping and expansion of the stent. Again, there was no visible delamination or cracking of the TiO₂ coating layer. Note that the TiO₂ coating layer in FIG. 9 is so thin that the paclitaxel particles are visible through the coating layer.

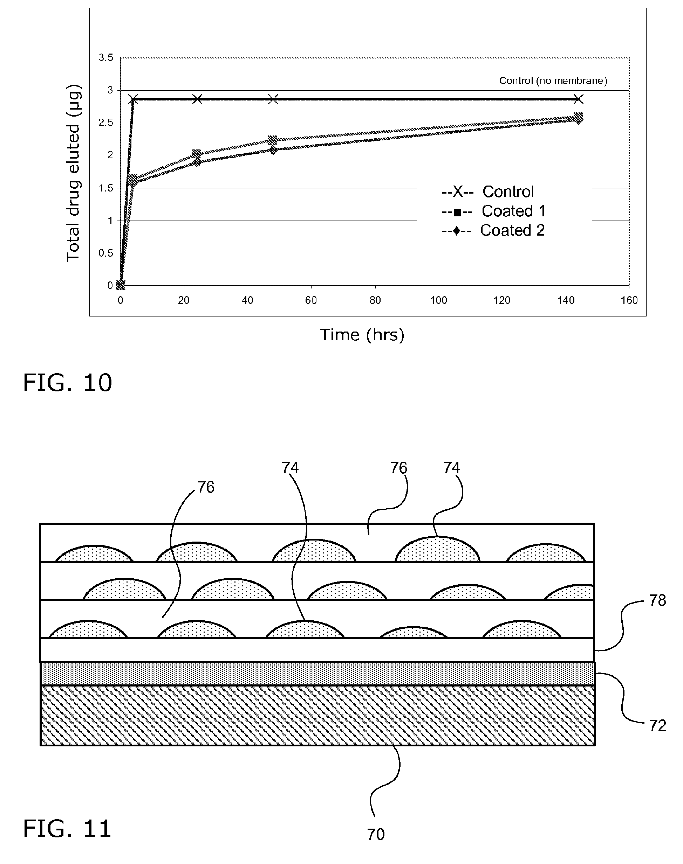

FIG. 10 shows the results of experiments testing the barrier characteristics of an inorganic coating layer deposited by atomic layer deposition. A 50 nm Al₂O₃ coating layer was deposited by atomic layer deposition onto paclitaxel-coated stents. The Al₂O₃-coated stents, along with a bare paclitaxel-coated stent (not covered by an inorganic coating layer) as a control, were immersed in an aqueous saline solution and the amount of drug eluted was measured over time. For the bare paclitaxel-coated stent, there was a nearly immediate release of substantially all the drug. In comparison, for the Al₂O₃-coated stents, there was some immediate release of the drug, and then a more prolonged course of drug release over a period of about one week. This prolonged course of drug release is believed to involve dissolution of the Al₂O₃ coating layer in the saline solution.

In certain embodiments, the coating on the medical device comprises a plurality of inorganic coating layers. Using multiple inorganic coating layers may allow for an additional degree of control in varying the properties of the coating. For example, multiple inorganic coating layers may be designed to cooperate with each other in controlling the release of therapeutic agent. Referring to the embodiment shown in FIG. 11, a multilayered coating is provided on a portion 70 of a medical device. The multilayered coating comprises a therapeutic agent layer 72 and multiple inorganic coating layers over the therapeutic agent. A 15 nm Al₂O₃ layer 78 is deposited onto therapeutic agent layer 72. Next, a very thin 0.5 nm TiO₂ layer 74 is deposited on the Al₂O₃ layer 78. This TiO₂ layer 74 is so thin that the layer is discontinuous and only islands of coating are formed. A 1 nm thick Al₂O₃ layer 76 is then deposited on the TiO₂ layer 74. This process is repeated to form alternating Al₂O₃ and TiO₂ layers over therapeutic agent layer 72.

In certain embodiments, in addition to the inorganic coating layer over the therapeutic agent, the coating of the present disclosure further comprises a base coating layer that is underneath the inorganic coating layer. The base coating layer may be in contact with the therapeutic agent or disposed between the surface of the medical device and the therapeutic agent (i.e., at least a portion of the base coating layer is underneath the therapeutic agent). This base coating layer may serve various functions, including serving as a carrier for the therapeutic agent, improving the adhesion of the therapeutic agent with the surface of the medical device, controlling the release of the therapeutic agent, or providing a biocompatible surface for the medical device after the inorganic coating layer is degraded and the therapeutic agent is released. This base coating layer may be located directly on a surface of the medical device or otherwise over a surface of the medical device (i.e., there may be intervening layers between the surface of the medical device and the base coating layer).

The base coating layer may be formed by a self-limiting deposition process of the present disclosure (e.g., by atomic layer deposition) or any other suitable coating technique. For example, the base coating layer may be applied by high speed impaction of inorganic particles, as described in U.S. Provisional Application Serial No. 61/047,495 entitled "Medical Devices Having Inorganic Particle Layers" (filed on 24 April 2008, by Kuchling et al.), which is incorporated by reference herein. The base coating layer may be inorganic or organic (e.g., polymeric). In some cases, the base coating layer may serve as a carrier for the therapeutic agent. For example, the base coating layer may comprise porous nanoparticles, with the therapeutic agent contained in the porous nanoparticles. In another example, the therapeutic agent may be coated over the nanoparticles. In some cases, the base coating layer has a thickness of less than 30 nm, and may be as thin as 0.5 nm, but other thicknesses are also possible.

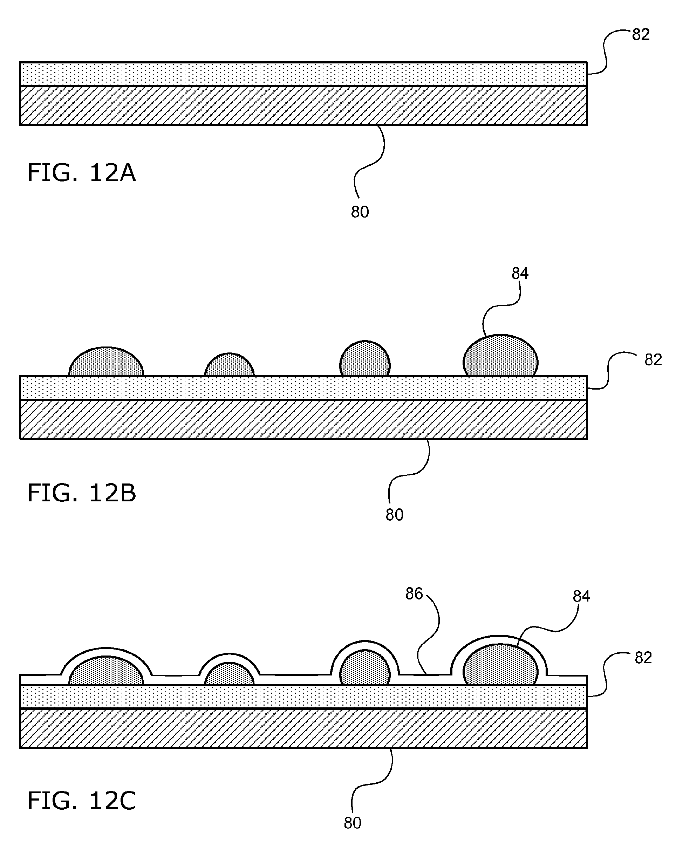

Referring to the embodiment shown in FIGS. 12A - C, a portion 80 of a medical device is coated with a titanium oxide base coating layer 82 deposited by atomic layer deposition. Base coating layer 82 is then spray-coated with a therapeutic agent, which forms particles 84 of therapeutic agent on base coating layer 82. A nanoporous aluminum oxide coating layer 86 (i.e., a barrier layer) is then formed over particles 84 and base coating layer 82 by atomic layer deposition.

Upon implantation of the medical device in a patient's body, there may be an initial burst release of the therapeutic agent through the nanoporous aluminum oxide coating layer 86. Over a longer period of time, there is continued release of the therapeutic agent by diffusion through inorganic coating layer 86 as well as by degradation of inorganic coating layer 86. Degradation of inorganic coating layer 86 and release of the therapeutic agent continues until the titanium oxide base coating layer 82 is remaining. The titanium oxide base coating layer 82 that remains provides a biocompatible surface for the medical device.

In certain embodiments, the coating on the medical device is essentially free of any polymeric material (excluding the presence of any small amounts of polymeric materials that may have been introduced incidentally during the manufacturing process such that someone of ordinary skill in the art would nevertheless consider the coating to be free of any polymeric material).

In certain embodiments, the inorganic coating layer may comprise a material that is capable of undergoing a photocatalytic effect such that the coating becomes superhydrophilic. For example, titanium oxide coatings can be made superhydrophilic and/or hydrophobic using the technique described in U.S. Patent Application Publication No. 2008/0004691 titled "Medical Devices With Selective Coating" (by Weber et al., for Application Serial No. 11/763,770), which is incorporated by reference herein. For example, after a titanium oxide coating layer is applied over a medical device, the medical device can be placed in a dark environment to cause the titanium oxide coating layer to become hydrophobic, followed by exposure of the coating layer (or selected portions of the coating layer) to UV light to cause the coating layer (or selected portions) to become superhydrophilic (i.e., such that a water droplet on the coating layer would have a contact angle of less than 5°). Superhydrophilic coating layers can be useful for carrying therapeutic agents, providing a more biocompatible surface for the medical device, and/or promoting adherence of endothelial cells to the medical device.

By selectively making some portions of the coating layer more hydrophilic or hydrophobic relative to other portions, it may be possible to selectively apply other materials, such as drugs or other coating materials, onto the medical device based on the hydrophilicity or hydrophobicity of these other materials. For example, referring back to FIG. 3B, the inner coating layer 44 can be made superhydrophilic by UV light exposure through a fiber optic line inserted within the lumen 46 of stent 40, or the external coating layer 52 can be made superhydrophilic by exposing the exterior of stent 40 to UV light. A hydrogel coating containing a therapeutic agent can then be applied onto the superhydrophilic portions of the coating layer.

Non-limiting examples of medical devices that can be used with the present invention include stents, stent grafts, catheters, balloon catheters, guide wires, neurovascular aneurysm coils, balloons, filters (*e.g.,* vena cava filters), vascular grafts, intraluminal paving systems, pacemakers, electrodes, leads, defibrillators, joint and bone implants, spinal implants, access ports, intra-aortic balloon pumps, heart valves, sutures, artificial hearts, neurological stimulators, cochlear implants, retinal implants, and other devices that can be used in connection with therapeutic coatings. Such medical devices are implanted or otherwise used in body structures, cavities, or lumens such as the vasculature, gastrointestinal tract, abdomen, peritoneum, airways, esophagus, trachea, colon, rectum, biliary tract, urinary tract, prostate, brain, spine, lung, liver, heart, skeletal muscle, kidney, bladder, intestines, stomach, pancreas, ovary, uterus, cartilage, eye, bone, joints, and the like. The stent may be any of those known in the art, including those that are biostable (e.g., made of stainless steel), bioerodable (e.g., made of magnesium), or biodegradable.

The therapeutic agent used in the present invention may be any pharmaceutically acceptable agent (such as a drug), a biomolecule, a small molecule, or cells. Exemplary drugs include anti-proliferative agents such as paclitaxel, sirolimus (rapamycin), tacrolimus, everolimus, biolimus, and zotarolimus. Exemplary biomolecules include peptides, polypeptides and proteins; antibodies; oligonucleotides; nucleic acids such as double or single stranded DNA (including naked and cDNA), RNA, antisense nucleic acids such as antisense DNA and RNA, small interfering RNA (siRNA), and ribozymes; genes; carbohydrates; angiogenic factors including growth factors; cell cycle inhibitors; and anti-restenosis agents. Exemplary small molecules include hormones, nucleotides, amino acids, sugars, and lipids and compounds have a molecular weight of less than 100kD. Exemplary cells include stem cells, progenitor cells, endothelial cells, adult cardiomyocytes, and smooth muscle cells.

The foregoing description and examples have been set forth merely to illustrate the invention and are not intended to be limiting. Each of the disclosed aspects and embodiments of the present invention may be considered individually or in combination with other aspects, embodiments, and variations of the invention. In addition, unless otherwise specified, the steps of the methods of the present invention are not confined to any particular order of performance. Modifications of the disclosed embodiments incorporating the spirit and substance of the invention may occur to persons skilled in the art, and such modifications are within the scope of the present invention.

The invention is summarized by the following items :
1. A medical device having a coating, the coating comprising:
   an inorganic coating layer comprising an inorganic material, wherein the inorganic coating layer has a thickness of less than 30 nm.
2. The medical device of item 1, wherein the inorganic coating layer conformally coats over the medical device.
3. The medical device of item 1, wherein the medical device is a stent, and wherein the thickness of the inorganic coating layer on the luminal surface of the stent and the thickness of the inorganic coating layer on the external surface of the stent are substantially the same or differ by less than 20% of the thickness of the inorganic coating layer on the external surface of the stent.
4. The medical device of item 1, wherein the inorganic coating layer is formed by atomic layer deposition.
5. The medical device of item 1, wherein the coating further comprises a therapeutic agent, and wherein the inorganic coating layer covers the therapeutic agent.
6. The medical device of item 5, wherein at least 50% of the therapeutic agent is eluted in 7 days after immersion in an aqueous solution or after implantation in a human body.
7. The medical device of item 5, wherein the therapeutic agent is distributed as particles, and wherein the inorganic coating layer conformally coats over the particles of therapeutic agent.
8. The medical device of item 5, further comprising a base coating layer disposed beneath the inorganic coating layer, wherein the base coating layer is in contact with the therapeutic agent or disposed between the therapeutic agent and the surface of the medical device.
9. The medical device of item 8, wherein the inorganic coating layer comprises aluminum oxide and wherein the base coating layer comprises titanium oxide.
10. The medical device of item 1, wherein the coating is essentially free of any polymeric material.
11. A method of coating a medical device, comprising:
   providing a medical device having a coating of therapeutic agent; and
   depositing an inorganic coating layer over the therapeutic agent by atomic layer deposition.
12. The method of item 11, wherein the inorganic coating layer is deposited to a thickness of less than 30 nm.
13. The method of item 11, wherein depositing the inorganic coating layer is performed at a deposition temperature of less than 125 °C.
14. The method of item 11, wherein providing the medical device comprises:
   depositing a base coating layer over the medical device; and
   depositing the therapeutic agent over the base coating layer.
15. The method of item 11, wherein the inorganic coating layer comprises an inorganic oxide.
16. A method of medical treatment comprising:
   providing a medical device having a coating comprising an inorganic coating layer, the inorganic coating layer comprising an inorganic material;
   implanting the medical device into the patient's body; and
   deforming the medical device without substantially cracking or delaminating the inorganic coating layer.
17. The method of item 16, wherein the coating further comprises a therapeutic agent and the inorganic coating layer covers over the therapeutic agent, and wherein the method further comprises degrading the inorganic coating layer after implanting the medical device into the patient's body.
18. The method of item 17, further comprising eluting at least 50% of the therapeutic agent within 7 days after implanting the medical device.
19. The method of item 17, further comprising degrading the inorganic coating layer completely within 4 weeks after implanting the medical device.
20. The method of item 16, wherein the medical device is a stent, and wherein deforming the stent comprises expanding the stent such that the stent diameter increases by at least 50%.
21. A stent having a luminal surface, an exterior surface, and a coating, the coating comprising:
   an inner inorganic coating layer disposed over the luminal surface of the stent; and
   an external inorganic coating layer disposed over the external surface of the stent;
   wherein the thickness of the inner coating layer and the thickness of the external coating layer are substantially the same or differ by less than 20% of the thickness of the external coating layer.
22. The stent of item 21, wherein the coating is conformal over the stent.
23. The stent of item 21, wherein the inner inorganic coating layer has a thickness of less than 30 nm.
24. A medical device having a coating, the coating comprising:
   a therapeutic agent; and
   an inorganic coating layer disposed over the therapeutic agent, wherein the inorganic coating layer is formed by atomic layer deposition.
25. The medical device of item 24, wherein the inorganic coating layer is porous to the therapeutic agent.
26. The medical device of item 24, further comprising a base coating layer disposed beneath the inorganic coating layer, wherein the base coating layer is in contact with the therapeutic agent or disposed between the surface of the medical device and the therapeutic agent.
27. The medical device of item 24, wherein the inorganic coating layer comprises aluminum oxide and the base coating layer comprises titanium oxide.

## Claims

1. A stent having a coating, the coating comprising a therapeutic agent and an inorganic coating layer comprising an inorganic material;
wherein the inorganic coating layer has a thickness of less than 30 nm; wherein the inorganic coating layer is formed by atomic layer deposition and conformally coats over the medical device and wherein the thickness of the inorganic coating layer on the luminal surface of the stent and the thickness of the inorganic coating layer on the external surface of the stent are substantially the same or differ by less than 20% of the thickness of the inorganic coating layer on the external surface of the stent; and
wherein the therapeutic agent is distributed as particles and wherein the inorganic coating layer conformally coats over the particles of therapeutic agent.

2. The stent of claim 1, wherein the coating is essentially free of any polymeric material.

3. The stent of claim 1, wherein the inorganic coating layer is porous to the therapeutic agent.

4. The stent of claim 1, wherein the inorganic coating layer has a thickness of less than 20 nm.

5. The stent of claim 1, wherein the inorganic material is titanium oxide, aluminum oxide, silicon oxide, or zinc oxide.

6. The stent of claim 1, wherein the inorganic material is titanium oxide.

7. The stent of claim 1, wherein the inorganic material is aluminum oxide.

8. method of preparing the stent of any one of claims 1 to 7, comprising:
providing a stent having a particulate coating of a therapeutic agent; and
depositing an inorganic coating layer over the particles of the therapeutic agent by atomic layer deposition.

## Patentansprüche

1. Stent mit einer Beschichtung, wobei die Beschichtung ein therapeutisches Mittel und eine anorganische Beschichtungsschicht umfasst, die ein anorganisches Material umfasst;
wobei die anorganische Beschichtungsschicht eine Dicke von weniger als 30 nm aufweist;
wobei die anorganische Beschichtungsschicht durch Atomlagenabscheidung gebildet ist und konform über die medizinische Vorrichtung geschichtet ist und wobei die Dicke der anorganischen Beschichtungsschicht auf der luminalen Oberfläche des Stents und die Dicke der anorganischen Beschichtungsschicht auf der externen Oberfläche des Stents im Wesentlichen gleich sind oder sich um weniger als 20 % der Dicke der anorganischen Beschichtungsschicht auf der externen Oberfläche des Stents unterscheiden; und
wobei das therapeutische Mittel als Partikel verteilt ist und wobei die anorganische Beschichtungsschicht konform über die Partikel des therapeutischen Mittels geschichtet ist.

2. Stent gemäß Anspruch 1, wobei die Beschichtung im Wesentlichen frei von jeglichem Polymermaterial ist.

3. Stent gemäß Anspruch 1, wobei die anorganische Beschichtungsschicht für das therapeutische Mittel durchlässig ist.

4. Stent gemäß Anspruch 1, wobei die anorganische Beschichtungsschicht eine Dicke von weniger als 20 nm aufweist.

5. Stent gemäß Anspruch 1, wobei das anorganische Material Titanoxid, Aluminiumoxid, Siliciumoxid oder Zinkoxid ist.

6. Stent gemäß Anspruch 1, wobei das anorganische Material Titanoxid ist.

7. Stent gemäß Anspruch 1, wobei das anorganische Material Aluminiumoxid ist.

8. Verfahren zum Herstellen des Stents gemäß einem der Ansprüche 1 bis 7, umfassend:
Bereitstellen eines Stents mit einer Partikulatbeschichtung aus einem therapeutischen Mittel; und
Abscheiden einer anorganischen Beschichtungsschicht über die Partikel des therapeutischen Mittels durch Atomlagenabscheidung.

## Revendications

1. Endoprothèse ayant un revêtement, le revêtement comprenant un agent thérapeutique et une couche de revêtement inorganique comprenant un matériau inorganique ;
dans laquelle la couche de revêtement inorganique a une épaisseur inférieure à 30 nm ;
dans laquelle la couche de revêtement inorganique est formée par déposition de couche atomique et revêt le dispositif médical en s'y conformant, et dans laquelle l'épaisseur de la couche de revêtement inorganique sur la surface luminale de l'endoprothèse est pratiquement identique à l'épaisseur de la couche de revêtement inorganique sur la surface externe de l'endoprothèse ou diffère de moins de 20 % de l'épaisseur de la couche de revêtement inorganique sur la surface externe de l'endoprothèse ; et
dans laquelle l'agent thérapeutique est distribué sous la forme de particules et dans laquelle la couche de revêtement inorganique revêt en s'y conformant les particules de l'agent thérapeutique.

2. Endoprothèse selon la revendication 1, dans laquelle le revêtement est pratiquement exempt de tout matériau polymère.

3. Endoprothèse selon la revendication 1, dans laquelle la couche de revêtement inorganique est poreuse vis-à-vis de l'agent thérapeutique.

4. Endoprothèse selon la revendication 1, dans laquelle la couche de revêtement inorganique a une épaisseur inférieure à 20 nm.

5. Endoprothèse selon la revendication 1, dans laquelle le matériau inorganique est l'oxyde de titane, l'oxyde d'aluminium, l'oxyde de silicium, ou l'oxyde de zinc.

6. Endoprothèse selon la revendication 1, dans laquelle le matériau inorganique est l'oxyde de titane.

7. Endoprothèse selon la revendication 1, dans laquelle le matériau inorganique est l'oxyde d'aluminium.

8. Procédé pour préparer l'endoprothèse de l'une quelconque des revendications 1 à 7, comprenant les opérations consistant à :
fournir une endoprothèse ayant un revêtement particulaire d'un agent thérapeutique ; et
déposer une couche de revêtement inorganique sur les particules de l'agent thérapeutique par déposition de couche atomique.
